Europäisches Patentamt

European Patent Office

Office européen des brevets

(11) Publication number : **0 519 728 A2**

# EUROPEAN PATENT APPLICATION

(21) Application number : 92305627.9

(22) Date of filing : 19.06.92

(51) Int. Cl.⁵ : **C12P 21/08**, G01N 33/577, G01N 33/68, // C07K3/18

(30) Priority : **21.06.91 JP 177236/91**

(43) Date of publication of application :
**23.12.92 Bulletin 92/52**

(84) Designated Contracting States :
**AT BE CH DE DK ES FR GB IT LI NL SE**

(71) Applicant : **SNOW BRAND MILK PRODUCTS & CO., LTD.**
**1-1, Naebo-cho 6-chome Higashi-ku**
**Sapporo-shi Hokkaido 065 (JP)**

(72) Inventor : **Higashio, Kanji**
**1769-10, Yamada**
**Kawagoe-shi, Saitama (JP)**
Inventor : **Itagaki, Yasuharu**
**1-27, Minami Iljo Nishi 18-chome**
**Chuo-ku, Sapporo-shi (JP)**
Inventor : **Ohgaki, Fumiko**
**8-11, Yamato 1-chome**
**Utsunomiya-shi, Tochigi (JP)**

(74) Representative : **Davies, Jonathan Mark**
**Reddie & Grose 16 Theobalds Road**
**London WC1X 8PL (GB)**

(54) **Anti-TCF-II monoclonal antibodies and method for the measurement of TCF-II by applying the antibodies.**

(57)    Disclosed are monoclonal antibodies having specific affinity to an anti-tumor protein, TCF-II, derived from human fibroblasts. The monoclonal antibodies have a molecular weight of approximately 150,000 and belong to a subclass of $IgG_1$, with N-terminal amino acid sequence of the L chain identified. The monoclonal antibodies can be used for the purification of TCF-II and for the diagnosis of liver disease by measuring the content of TCF-II in the plasma by the aid of the monoclonal antibodies.

EP 0 519 728 A2

Jouve, 18, rue Saint-Denis, 75001 PARIS

## BACKGROUND OF THE INVENTION

### 1. Field of the Invention

The present invention relates to novel monoclonal antibodies to a human anti-tumor protein, termed TCF-II, and a method for the measurement of TCF-II by employing the monoclonal antibodies.

### 2. Description of the Related Art

β-Interferon is well known as a tumor cytotoxic factor, which is produced by human fibroblasts. Other substances produced by the fibroblasts are disclosed, respectively, in Japanese Patent Laid-open Publication (kokai) Nos. 58-146,293, 61-33,120, 61-1,872, 62-103,021 and 64-10,998. In the process of the investigation of anti-tumor proteins derived from the human fibroblasts, the present inventors have found a novel anti-tumor protein different from those having so far been reported and succeeded in the cloning of a cDNA encoding this protein. Further, they have determined all the primary amino acid sequence of this protein and confirmed its utility. This novel anti-tumor protein and its gene are disclosed in WO90/10651, and the novel anti-tumor protein is termed as TCF-II. All the primary amino acid sequences of the anti-tumor protein, TCF-II, which is deduced from its cDNA, is indicated in Table 1.

It is further confirmed that the TCF-II has a strong anti-tumor activity and a growth stimulating activity for normal cells and that it is a kind of families of hepatocyte growth factors, HGFs, which are potent growth stimulators for hepatocytes. The TCF-II has the molecular weight (MW) of $78,000 \pm 2,000$ or $74,000 \pm 2,000$, on SDS-polyacrylamide gel electrophoresis under non-reducing conditions. Under the non-reducing conditions, it separates into three polypeptide chains: A-chain, as a common band, with the MW of $52,000 \pm 2,000$; B-chain with the MW of $30,000 \pm 2,000$; and C-chain with the MW of $26,000 \pm 2,000$. The N-terminus of the A-chain is blocked. The B- and C-chains have the same N-terminal amino acid sequence. The TCF-II essentially has a heterodimer structure composed of the A-chain and B-chain or C-chain.

It is further to be noted that neither monoclonal nor polyclonal antibodies to the TCF-II have been produced.

With attention paid to the utility of the TCF-II, the present inventors have investigated the utilization of the TCF-II as a anti-tumor agent or a diagnostic marker. In order to quantitate the TCF-II, there has so far been employed only biological assay method that utilizes the cytotoxic effect on tumor cells. Immunological assay is currently taking the main place due to accuracy of the determination of such a substance as being contained in a minute amount, so that strong demands have been made to develop an antibody capable of being employed for the measurement of the TCF-II. It is further noted that, although the TCF-II can be purified by repeating classical gel filtration and adsorptive chromatographies, the use of the antibody can remarkably improve the efficiency of purifying and recovering the TCF-II by taking advantage of affinity chromatography using such antibodies.

## SUMMARY OF THE INVENTION

Therefore, the present invention has the object to provide monoclonal antibodies having high specificity and high affinity to TCF-II.

Another object of the present invention is to provide a method for measuring the TCF-II by employing the monoclonal antibodies.

## BRIEF DESCRIPTION OF THE DRAWINGS

Fig. 1 shows standard curves for measuring TCF-II, being obtainable in various combination of the monoclonal antibodies.

Fig. 2 shows the results of recovery of the TCF-II added to the plasma and specificity of the monoclonal antibodies, used in the measurement system, to the TCF-II.

Fig. 3 shows the results of measurement for the contents of TCF-II in the plasma samples from patients with liver diseases.

Fig. 4 shows changes in the TCF-II levels in the patients with acute hepatitis at acute stage and at the recovery stage.

## DESCRIPTION OF THE PREFERRED EMBODIMENTS

The monoclonal antibodies according to the present invention indicate specific affinity to human anti-tumor protein termed TCF-II. The antibodies have a molecular weight of about 150,000 and belong to IgG$_1$ subclass. Further, each monoclonal antibody has an N-terminal amino acid sequence of its L chain defined by either one of the amino acid sequences (1) to (3), inclusive, as follows:

Amino acid sequence (1):

Asp-Val-Val-Leu-Thr-Gln-Ser-Pro-Ala-

1    2    3    4    5    6    7    8    9

Thr-Leu-Ser-Val-Thr-Pro-Gly-Asp-Ser-

10   11   12   13   14   15   16   17   18

Val

19

Amino acid sequence (2):

Asp-Ser-Val-Leu-Thr-Gln-Ser-Pro-Ala-

1    2    3    4    5    6    7    8    9

Ser-Leu-Ala-Val-Ser-Leu-Gly-Gln-Arg-

10   11   12   13   14   15   16   17   18

Ala-Thr-Ile-Ser

19   20   21   22

Amino acid sequence (3):

Asp-Ile-Val-Leu-Thr-Gln-Ser-Pro-Val-

1    2    3    4    5    6    7    8    9

Thr-Leu-Ser-Val-Thr-Pro-Gly-Gly-Ser-

10   11   12   13   14   15   16   17   18

Val

19

The monoclonal antibody according to the present invention can be prepared in the manner as will be described hereinafter. The monoclonal antibody can be prepared in accordance with conventional procedures by employing TCF-II as an antigen. The TCF-II can be produced from human fibroblasts according to the procedures as described in WO90/10651 or from microorganisms or other cells by recombinant technique on the basis of the cDNA sequence disclosed in WO90/10651. Further, there also can be employed synthetic peptides synthesized on the basis of the amino acid sequence of the TCF-II or peptides derived from partially decomposed TCF-II. It is also to be noted that these antigens may not necessarily be purified homogeneously. The antigen is applied to immunize mammalian animals in vivo or lymphocytes (splenocytes) in vitro, and then the splenocytes are fused with myeloma cells derived from a mammalian animal. The fusion suspension is distributed into 96-well plates and cultured in medium containing HAT (hypoxanthine, amino protein, and thymidine). After 7-10 days, the plates are examined for the growth of hybridomas. The supernatant removed from these cells was screened for the presence of anti-TCF-II antibodies by ELISA. The hybrid cells which produce the desired antibody are cloned by limiting dilution and the clones are assayed again. The established hybridomas may be cultured in flasks or grown in mice to produce the objective antibodies.

In employing the mammalian animals in the formation of the hybridomas, it is common to use a small animal such as mouse or rat although there may be employed any mammalian animals.

Immunization may be carried out by diluting the TCF-II with physiological saline to appropriate concentrations, administering the small animals such as mice or rats with a given quantity of the resulting solution through intravenous or intraperitoneal route two to five times at every two to twenty days. An adjuvant is administered, if needed or when desired, in combination with a TCF-II antigen. The animals are then sacrificed in the third day after final immunization and the spleens are taken out from the animals. The splenocytes are fused with a mouse myeloma cell line, for example, p3/x63-Ag8, p3-U1, NS-1, MPC-11, SP2/0, FO, P3x63Ag8.653, S194 and so on. Rat-derived cells may include cell lines such as R-210. A humanized antibody may be produced by immunizing human B lymphocytes in vitro and employing human myeloma cell lines or human B cell lines transformed by EB viruses as a parent cell line.

The fusion of the splenocytes with the myeloma cells may be carried out in conventional manner, as disclosed, for example, in Koehler, G., et al.: Nature, Vol. 256, 11 495-497, 1975. An electric pulse method may also be employed. The splenocytes are mixed with the myeloma cells in a conventional ratio, and the mixture is incubated for fusion in a serum-free medium which is conventionally employed for cell culture, to which polyethylene glycol has been added. The fusion suspension is distributed into 96-well plates and cultured in HAT medium containing FCS to select fused cells.

For the screening of the cells capable of producing anti-TCF-II antibodies, there may be employed procedures as have been conventionally employed for detection of antibodies, such as ELISA, plaque, ouchterlony, aggregation or the like. Among them, the ELISA method using purified TCF-II can select the desired antibody-producible cells with comparatively easy and high accuracy.

The hybridomas selected in the manner as described above can be sub-cultured in conventional manner and may be frozen for storage as needed. The hybridomas may be incubated in conventional manner and transplanted peritoneally into mice. The ascites containing anti-TCF-II antibodies are recovered from the mice and the antibodies in the ascites are purified by conventional method such as salting-out, gel filtration, affinity chromatography or the like.

The resulting antibody is found to react specifically with the TCF-II and can be employed for measurement and purification of the TCF-II. In using the antibody for the measurement of the TCF-II, the antibody is labelled with an isotope or an enzyme and can be employed as an antibody specific to the TCF-II-in the measurement system which is known as radioimmunoassay (RIA) or enzyme immunoassay (EIA). Since each antibody in the present invention recognizes the different sites in the molecule of the TCF-II, the antibodies-can be employed for sandwich immunoassay. The use of this assay system makes it possible to easily measure the amount of the TCF-II antigen in samples such as blood, urine and so on as well as a culture solution.

The TCF-II can readily be purified by means of immunoprecipitation or affinity chromatography prepared by immobilizing the antibody on a carrier such as Affigel 10 (Biorad).

The present invention will then be described more in detail by way of examples.

**Example 1**

**Purification of TCF-II for Antigen**

Purified TCF-II was prepared by incubating cells in accordance with the method as disclosed in WO90/10651 and the purification method as disclosed by Higashio, K. et al. (B.B.R.C., vol. 170, pp. 397-404, 1990).

A total number of $3 \times 10^6$ cells of human fibroblasts, IMR-90 (ATCC CCL 186), was seeded in a roller bottle (one liter; Corning) containing 100 ml of DMEM with 10% calf serum (Hyclone). The cells were cultured by rotating the roller bottle at 0.5 to 2 rpm for seven days. The cells were then tripsinized when the total cell numbers reached $1 \times 10^7$. The cells were suspended in 250 ml of DMEM with 10% calf serum and allowed to settle to the bottom of the bottle. Ceramic pieces (3.5 to 5.0 mesh; Toshiba Ceramics Co., Ltd.) were autoclaved and 100 grams of the autoclaved ceramic pieces were added to the cell suspension in the roller bottle as the cell matrices; static culture was continued for 24 hours. After the static culture at 37° C for 24 hours, the roller bottle was supplemented with 250 ml of the same medium and further incubated for 7 days. The culture medium was replaced with 500 ml of DMEM supplemented with 5% calf serum in every 7 to 10 days. The conditioned medium was collected during two months at a rate of four liters from every roller bottle.

The activity of the TCF-II in the collected conditioned medium was found to be 32 units per milliliter.

Seventy five liters of the culture medium were concentrated by treatment with ultrafiltration module (MW 6,000 cuts; Asahi Chemical Industries Co., Ltd.) in accordance with the method as described in Patent Publication No. 90/10651 (WO90/10651). The TCF-II in the concentrate was then purified by a four-step chromatography consisting of CM Sephadex C-50 (Pharmacia), ConA Sephadex (Pharmacia), Mono S column (Pharmacia) and Heparin Sepharose (Pharmacia), thereby yielding the purified TCF-II having the specific activity of 5,248,000 units per mg.

## Example 2

### Preparation of Hybridoma Producing Anti-TCF-II Antibody

The purified TCF-II obtained in Example 1 was dissolved in PBS at a concentration of 10 μg per 100 μl, and BALB/c mice were immunized with the resulting solution at every two weeks. At the first and second immunization, a mixture of Freund's complete adjuvant and the TCF-II solution (1 : 1) was administered to the mice. In the third day after final immunization, the spleens of the mice were taken out and B lymphocytes were isolated from the spleens. Then, the resulting B lymphocytes were fused with mouse myeloma cells, P3x63-AG8.653, in accordance with the method as described by Koehler, G., et al. (Nature: vol. 256, pp. 495-497, 1975). The fused cells were cultured in a HAT culture medium. In order to select hybridomas producing the antibody specific to the TCF-II, a supernatant of hybridoma culture was measured for its antibody specific to the TCF-II by means of solid phase ELISA using a microplate coated with the TCF-II. The hybridoma which had produced the objective antibody was cloned five or six times by the limiting dilution technique, while the quantity of the antibody produced by the cloned hybridoma was measured at every time by means of the ELISA.

Then, the clones which have high productivities of the antibodies were selected from the cloned hybridomas.

## Example 3

### Production of Monoclonal Antibody

An amount of 1 x 10⁶ cells of the strain producing a high amount of the antibody, obtained in Example 2, was intraperitoneally transplanted into Balb/c mice which were primed with pristane (Aldrich Chemicals, Inc.). In two weeks after transplantation, the accumulated ascites were collected, thereby producing the ascites containing the monoclonal antibodies according to the present invention. The antibody in the ascites was purified by means of Affigel Protein A Sepharose® (BioRad) chromatography in accordance with its manual. In other words, the ascites was diluted with the equal amount of a binding buffer (BioRad), and the resulting dilution was charged onto a protein A column, followed by washing the column with a sufficient amount of the binding buffer. The antibody, IgG, was eluted with an elution buffer (BioRad) and, after neutralization, the resulting eluate was then dialyzed against water, followed by lyophilization. The purified antibody was then subjected to SDS-PAGE electrophoresis to check its purity and it was found that the purified antibody migrates as a homogeneous band having a molecular weight of approximately 150,000.

## Example 4

### Selection of Monoclonal Antibody Having High Affinity to TCF-II

The antibodies obtained in Example 3 were dissolved in PBS and the concentration of the protein was determined by the Lowry method. Then, each of the antibodies was dissolved in PBS to give the same concentration. The resulting antibody solution was diluted by means of stepwise dilution, and the resulting antibody dilutions were measured by solid phase ELISA, as described in Example 2 above, to select the monoclonal antibody that can react with the TCF-II even at high dilution rates. As a result, three antibodies were selected and they were referred to as P5A8, P2D6 and P1C8. The strains producing the antibodies P5A8, P2D6 and P1C8 were referred to as P5A8 strain, P2D6 strain and P1C8 strain and deposited with under Deposit Nos. BP-3820, BP-3821 and BP-3822, respectively.

5

**Example 5**

**Analysis of Subclass of Antibody**

The subclass of each antibody selected in Example 4 was analyzed by an immunoglobulin subclass analysis kit (Funakoshi) in accordance with protocol as instructions as given on the kit. The analysis results are shown in Table 2.

T A B L E 2

| Name of Antibody | $IgG_1$ | $IgG_{2a}$ | $IgG_{2b}$ | $IgG_3$ | IgA | IgM |
|---|---|---|---|---|---|---|
| P5A8 | + | − | − | − | − | − |
| P2D6 | + | − | − | − | − | − |
| P1C8 | + | − | − | − | − | − |

As is apparent from Table 2, all the monoclonal antibodies were found positive to $IgG_1$ only, so that all the three monoclonal antibodies were confirmed that they belong to a subclass of $IgG_1$.

**Example 6**

**Determination of N-Terminal Amino Acid Sequence of Antibody**

It is well known that antibodies are proteins having the structure consisting of two chains, i.e. light and heavy chains. The two chains are classified into a heavy chain (a H chain) having a larger molecular weight and a light chain (an L chain) having a smaller molecular weight, and each of the chains of the monoclonal antibodies can be defined by their N-terminal amino acid sequences.

The N-terminal amino acid sequence of each antibody was determined in the manner as will be described hereinafter.

The purified antibody selected in Example 4 was dissolved at a final concentration of 2 μg/μl in 150 μl of SDS-PAGE buffer solution (a 10 mM Tris-hydrochloride buffer solution (pH 8.0) containing 1 mM of EDTA, 25% SDS, 0.01% BPB, 10% mercaptoethanol, and 10% glycerol). After the solution was heated at 100° C for 3 minutes, the antibody solution was centrifuged at 15,000 rpm for 3 minutes. The resulting supernatant was subjected to electrophoresis using a 10% SDS polyacrylamide gel. The H and L chains of the antibody were electrically transferred to a PVDF (polyvinylidene difluoride) membrane by the wester blotting method. The PVDF membrane with protein was stained with a Coomassie Brilliant Blue and the membrane with bands corresponding to the objective H and L chains were cut out. The cut membranes were introduced directly into vapor-phase protein sequencer (ABI) and subjected automatically to coupling, cleavage and conversion in accordance with the protocols, thereby yielding phenyl thiohydantoin (PTH) amino acid which in turn was dissolved in a 20% acetonitrile. The resulting solution was then applied to reverse-phase high-pressure liquid chromatography (ABI; C-18 column) and each PTH-amino acid was identified by comparing the retention time thereof with that of a standard PTH-amino acid. Although neither of the H chains of the three antibodies could be determined because their N-terminal amino acids were blocked, the L chains of the three antibodies were found to having the N-terminal amino acid sequences as follows:

Antibody P5A8:

Asp-Val-Val-Leu-Thr-Gln-Ser-Pro-Ala-
1    2    3    4    5    6    7    8    9 .

Thr-Leu-Ser-Val-Thr-Pro-Gly-Asp-Ser-
10  11  12  13  14  15  16  17  18

Val
19

Antibody P2D6:

Asp-Ser-Val-Leu-Thr-Gln-Ser-Pro-Ala-
1    2    3    4    5    6    7    8    9

Ser-Leu-Ala-Val-Ser-Leu-Gly-Gln-Arg-
10  11  12  13  14  15  16  17  18

Ala-Thr-Ile-Ser
19  20  21  22

Antibody P1C8:

Asp-Ile-Val-Leu-Thr-Gln-Ser-Pro-Val-
1    2    3    4    5    6    7    8    9

Thr-Leu-Ser-Val-Thr-Pro-Gly-Gly-Ser-
10  11  12  13  14  15  16  17  18

Val
19

## Example 7

### Measurement for TCF-II by ELISA

The best combination of the antibodies for sandwich ELISA was selected by using three antibodies P5A8, P2D6 and P1C8 as solid phase and labelled antibodies. The solid phase antibody of each antibody was prepared by dissolving the antibody at a final concentration of 10 μg per ml in a 0.1M sodium bicarbonate solution, pouring 100 μl of the solution into each well in 96-well microplates (NunC), allowing the microplates to stand overnight at room temperature. Subsequently, each well was filled with PBS containing a 1% BSA (bovine serum albumin), and then the microplates were allowed to stand at room temperature for one hour to block the residual binding sites on the plates; the microplates were washed three times with a washing buffer (PBS containing 0.05% Tween 20). On the other hand, each monoclonal antibody was labelled with peroxidase in accordance with the method devised by Ishikawa et al. (J. Immunoassay, vol. 4, pp. 209-327, 1983).

Samples of the TCF-II were prepared by diluting the TCF-II solution with a diluting buffer (PBS containing 0.0.1% BSA and 0.05% Tween® 20) and 100 µl of the sample was added to each well of the microplates. The microplates were allowed to stand at 37° C for 3 hours, followed by washing each well three times with the washing buffer, and adding 100 µl of the labelled antibody diluted 200- to 400-fold with the diluting buffer to each well. After the labelled antibody was added, the microplates were allowed to stand at 37° C for 2 hours and washed three times with the washing buffer, followed by adding 100 µl of a substrate solution (a 0.1 M citrate-phosphate buffer (pH 4.5) containing 0.4mg/ml of orthophenylenediamine hydrochloride and 0.006% hydrogen peroxide) to each well in the microplates. After the microplates were allowed to stand at 37° C in dark place for 30 minutes, 50 µl of 6N sulfuric acid was added to each well to stop enzymatic reaction and absorbency of each well at 492 nm was measured by microplate spectrophotometer (Corona).

The combinations between the solid phase antibodies and the labelled antibodies are as shown in Table 3.

<div align="center">

T A B L E  3

| Solid Phase Antibody | Labelled Antibody |
|:---:|:---:|
| P1C8 | P2D6 |
| P5A8 | P2D6 |
| P5A8 | P1C8 |

</div>

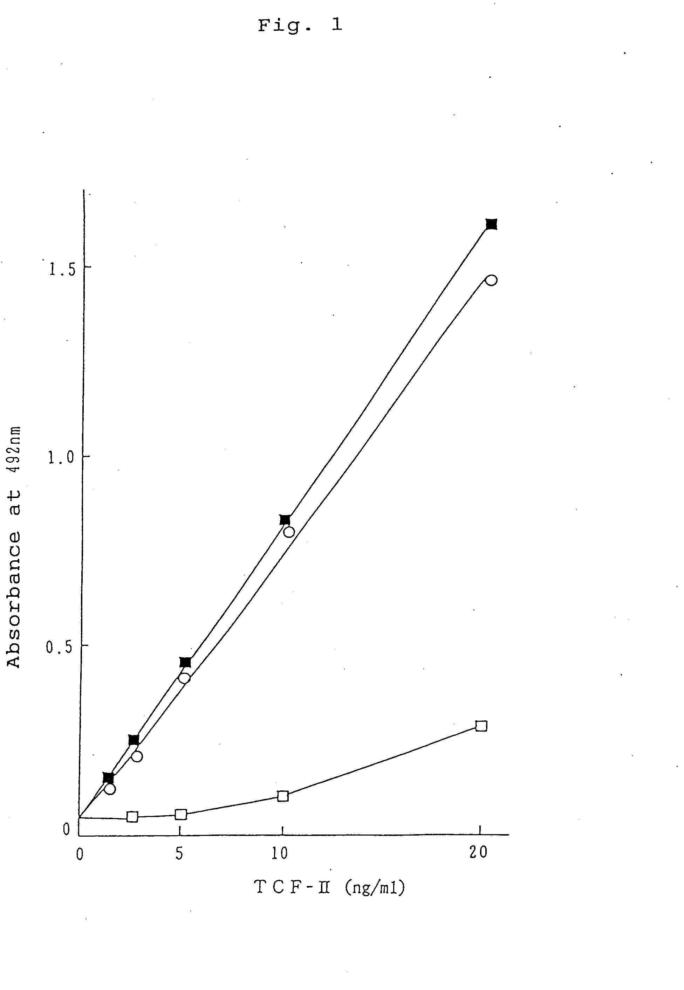

It can be noted that Fig. 1 shows the standard curves for measuring TCF-II, obtained at different combinations of the monoclonal antibodies. In Fig. 1, reference symbol "■-■" denotes a combination of the solid phase monoclonal antibody P1C8 and the labelled monoclonal antibody P2D6; reference symbol "○-○" denotes a combination of the solid phase monoclonal antibody P5A8 and the labelled monoclonal antibody P2D6; and reference symbol "□-□" denotes a combination of the solid phase monoclonal antibody P5A8 and the labelled monoclonal antibody P1C8. It can be found as a result that the measurement by the combination of the monoclonal antibodies P1C8 and P2D6 as well as that of the monoclonal antibodies P5A8 and P2D6 gave the good results.

It is further to be noted as apparent that each of the monoclonal antibodies P1C8, P5A8 and P2D6 has the property of recognizing epitopes which are different from each other.

## Example 8

### Determination of TCF-II in Human Plasma

The following tests were carried out using the combination of the solid phase monoclonal antibody P1C8 and the labelled monoclonal antibody P2D6.

1. Standard curve of TCF-II and recovery of added TCF-II in Plasma

As the solid phase antibody, the monoclonal antibody P1C8 was dissolved at a final concentration of 10 µg/ml in a 0.1 M sodium bicarbonate solution, and 100 µl of the solution was added to each well in 96-well microplates (NunC), followed by allowing the microplates to stand overnight at room temperature. The wells in the microplates were then filled with a solution which had been prepared by diluting Block Ace® (Snow Brand) twofold with distilled water in accordance with the procedures as described by Shinmoto et al. (Reports of Snow Brand Research Laboratories, vol. 88, pp. 45-51, 1989). The blocking was implemented by allowing the microplate to stand at room temperature for 1 hour, and the microplates were washed three times with a washing buffer (PBS containing 0.05% Tween® 20).

Then, the TCF-II samples having various concentrations were prepared by diluting the TCF-II solution with a diluting buffer (PBS containing 50% Block Ace® and 0.1% Tween® 20). To examine the recovery of the TCF-II from human plasma, samples of plasma containing exogenous TCF-II were prepared by adding the TCF-II having the same concentration series to human plasma. As a negative control, plasminogen, which is assumed to be high in structural homology to TCF-II (Nakamura et al.: Nature, vol. 34, pp. 440-443, 1989), was prepared by diluting the plasminogen solution with the same diluting buffer. A volume of 50 μl of the first reaction buffer (0.2M Tris-Hydrochloride buffer containing 50% Block Ace®, 0.2 M NaCl, 0.1% Tween® 20, 0.2% CHAPS, 20 mM benzamidine hydrochloride and 10 mM EDTA)were applied to each well in the microplates, and then a volume of 50 μl of each sample was applied to each well con 50 μl of the first buffer. The microplates were allowed to stand at 37° C for 3 hours, followed by washing three times with the washing buffer.

For the dilution of the labelled antibody, there was employed a 0.1 M phosphate buffer, pH 7, containing 1/10 Block Ace®, 0.15 M NaCl, 0.1% Tween® 20, 4% rat serum and 500 μg/ml of mouse IgG. To each well in the microplates was added 100 μl of a 400-fold dilution of the antibody P2D6 labelled with peroxidase. The microplates were allowed to stand at 37° C for 2 hours, followed by washing three times with the washing buffer and adding 100 μl of a substrate solution (a 0.1 M citrate-phosphate buffer, pH 4.5, containing 0.4 mg/ml of orthphenylenediamine hydrochloride and 0.006% hydrogen peroxide). The resulting microplates were then allowed to stand in dark place at 37° C for 30 minutes and 50 μl of 6 N sulfuric acid was added to each well in the microplates to cease the enzymatic reaction. Then, the absorbance at 492 nm of each well in the microplates was measured by the microplate spectrophotometer (corona).

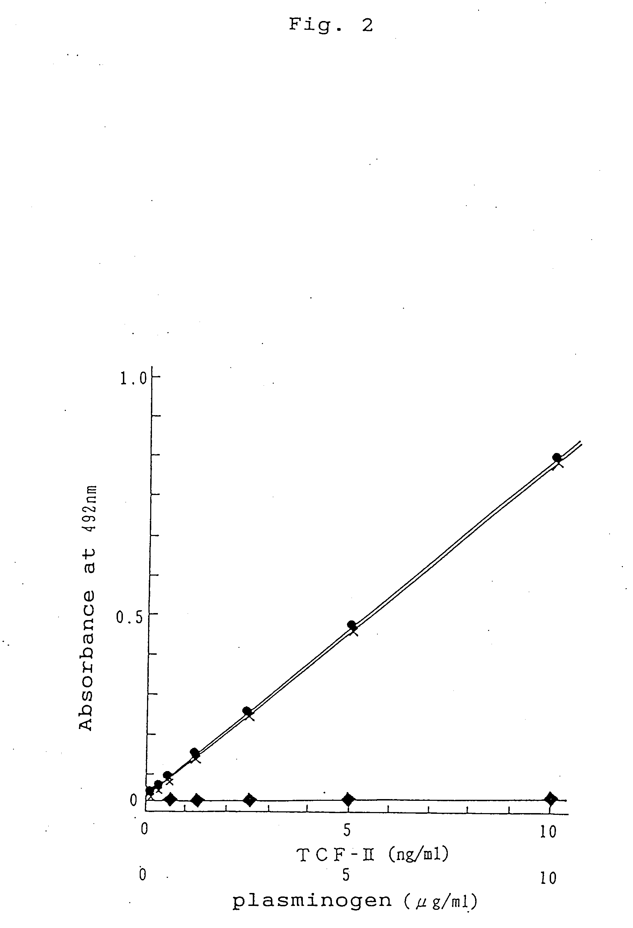

Fig. 2 shows the results of recovery of the TCF-II from the plasma and the specificity of the antibodies used in the assay system to the TCF-II. In Fig. 2, reference symbol "●-●" indicates the plasma samples to which the TCF-II was added; reference symbol "X-X" indicates standard TCF-II; reference symbol "♦-♦" indicates plasminogen.

As is apparent from Fig. 2, it is found that the sample with TCF-II added to the plasma indicates the line parallel to the standard curve of the TCF-II and it is thus confirmed that the whole amount of the TCF-II in the plasma has been recovered and that the plasminogen did not react at all in the measurement system.

2. Determination of TCF-II in the plasma samples from patients with liver diseases

The levels of the TCF-II in the plasma samples from 45 patients with liver diseases were determined by means of the system as described in item 1 above. The liver diseases include fulminant hepatitis, acute hepatitis, chronic active hepatitis, chronic inactive hepatitis, compensated liver cirrhosis, decompensated liver cirrhosis, and hepatocellular carcinoma.

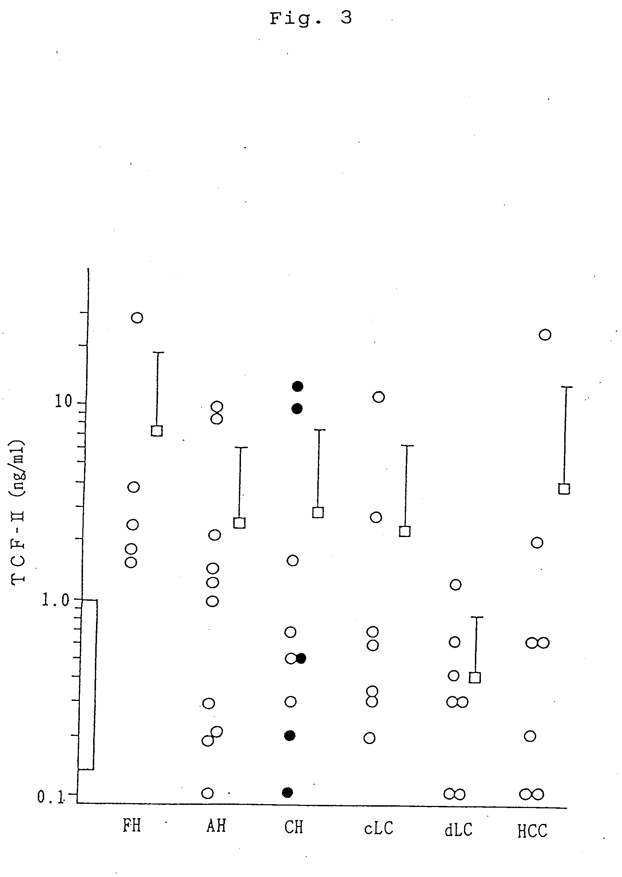

Fig. 3 indicates the results of determination of the TCF-II in the plasma from the patients with liver diseases. In Fig. 3, reference symbol "●" indicates a patient with chronic active hepatitis; reference symbol "□—┤" indicates an average and the range of ±1SD; reference symbol "□" indicates the range of the TCF-II for normal subjects. Fig. 3 indicates a normal control obtained from 21 healthy volunteers. Further, in Fig. 3, abbreviation "FH" means fulminant hepatitis; "AH" means acute hepatitis; "CH" means chronic hepatitis (inactive & active); "cLC" means compensated liver cirrhosis; "dLC" means a decompensated liver cirrhosis; and "HCC" means hepatocellular carcinoma. It is apparent from Fig. 3 that the plasma collected from the patients with liver diseases indicated a higher average concentration of the TCF-II than those collected from the normal persons.

3. Measurement for TCF-II in the plasma samples from the patients with acute hepatitis in the acute stage and in the recovery stage

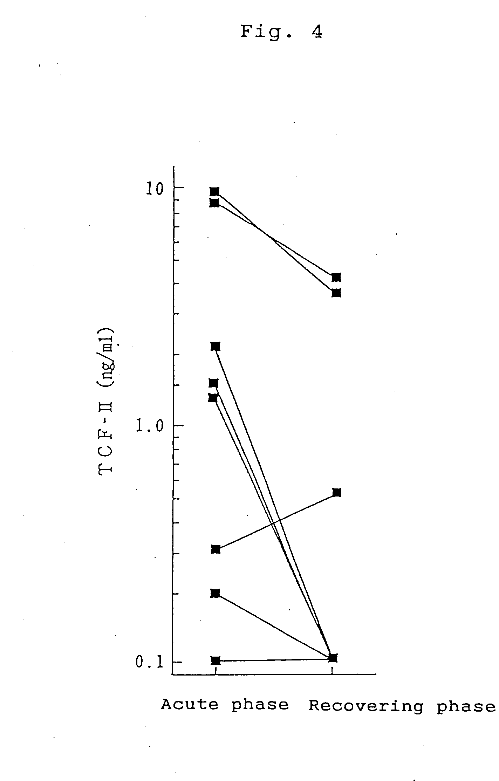

The levels of the TCF-II in the plasma samples from 8 patients with acute hepatitis were measured. The results of measurement are shown in Fig. 4. The results in Fig. 4 reveal that, except for one case, the levels of the TCF-II are reduced in the recovery stage of the acute hepatitis for the rest of the cases, so that it is recognized that the measurement of the TCF-II levels can serve as an indicator representing changes of the pathema of a patient with acute hepatitis.

As described hereinabove, the present invention can provide the monoclonal antibody to TCF-II, and the monoclonal antibody to the TCF-II is useful for the purification and determination of the TCF-II. The method for the determination of the TCF-II using the monoclonal antibodies according to the present invention can permit an accurate diagnosis of liver diseases or their pathemas by using an extremely small amount of samples.

SEQUENCE LISTING

Information for Seq. No: 1

Length: 19

Type: amino acids

Topology: linear

Molecular type: peptide

Sequence:

Asp Val Val Leu Thr Gln Ser Pro Ala Thr Leu Ser Val Thr Pro Gly
1                    5                    10                    15

Asp Ser Val
        19


Information for Seq. No:2

Length: 22

Type: amino acids

Topology: linear

Molecular type: peptide

Sequence:

Asp Ser Val Leu Thr Gln Ser Pro Ala Ser Leu Ala Val Ser Leu Gly
1                    5                    10                    15

Gln  Arg Ala Thr Ile Ser
            20      22

Information for Seq. No: 3

Length: 19

Type: amino acids

Topology: linear

Molecular type: peptide

Sequence:

Asp Ile Val Leu Thr Gln Ser Pro Val Thr Leu Ser Val Thr Pro Gly
1               5               10              15

Gly  Ser Val
              19


Information for Seq. No:4

Length: 723

Type: amino acids

Topology: linear

Molecular type: protein

Sequence:

Met Trp Val Thr Lys Leu Leu Pro Ala Leu Leu Leu Gln His Val Leu
1               5                10              15

Leu His Leu Leu Leu Leu Pro Ile Ala Ile Pro Tyr Ala Glu Gly Gln
              20                25              30

Arg Lys Arg Arg Asn Thr Ile His Glu Phe Lys Lys Ser Ala Lys Thr
              35                40              45

Thr Leu Ile Lys Ile Asp Pro Ala Leu Lys Ile Lys Thr Lys Lys Val
              50                55              60

Asn Thr Ala Asp Gln Cys Ala Asn Arg Cys Thr Arg Asn Lys Gly Leu
              65                70              75              80

Pro Phe Thr Cys Lys Ala Phe Val Phe Asp Lys Ala Arg Lys Gln Cys
                85                    90                    95

Leu Trp Phe Pro Phe Asn Ser Met Ser Ser Gly Val Lys Lys Glu Phe
                100                   105                  110

Gly His Glu Phe Asp Leu Tyr Glu Asn Lys Asp Tyr Ile Arg Asn Cys
                115                   120                  125

Ile Ile Gly Lys Gly Arg Ser Tyr Lys Gly Thr Val Ser Ile Thr Lys
                130                   135                  140

Ser Gly Ile Lys Cys Gln Pro Trp Ser Ser Met Ile Pro His Glu His
145                   150                   155                  160

Ser Tyr Arg Gly Lys Asp Leu Gln Glu Asn Tyr Cys Arg Asn Pro Arg
                165                   170                  175

Gly Glu Glu Gly Gly Pro Trp Cys Phe Thr Ser Asn Pro Glu Val Arg
                180                   185                  190

Tyr Glu Val Cys Asp Ile Pro Gln Cys Ser Glu Val Glu Cys Met Thr
                195                   200                  205

Cys Asn Gly Glu Ser Tyr Arg Gly Leu Met Asp His Thr Glu Ser Gly
                210                   215                  220

Lys Ile Cys Gln Arg Trp Asp His Gln Thr Pro His Arg His Lys Phe
225                   230                   235                  240

Leu Pro Glu Arg Tyr Pro Asp Lys Gly Phe Asp Asp Asn Tyr Cys Arg
                245                   250                  255

Asn Pro Asp Gly Gln Pro Arg Pro Trp Cys Tyr Thr Leu Asp Pro His
                260                   265                  270

Thr Arg Trp Glu Tyr Cys Ala Ile Lys Thr Cys Ala Asp Asn Thr Met
                275                   280                  285

Asn Asp Thr Asp Val Pro Leu Glu Thr Thr Glu Cys Ile Gln Gly Gln
                290                   295                  300

```
Gly Glu Gly Tyr Arg Gly Thr Val Asn Thr Ile Trp Asn Gly Ile Pro
305                 310               315                  320

Cys Gln Arg Trp Asp Ser Gln Tyr Pro His Glu His Asp Met Thr Pro
                325               330               335

Glu Asn Phe Lys Cys Lys Asp Leu Arg Glu Asn Tyr Cys Arg Asn Pro
                340               345               350

Asp Gly Ser Glu Ser Pro Trp Cys Phe Thr Thr Asp Pro Asn Ile Arg
                355               360               365

Val Gly Tyr Cys Ser Gln Ile Pro Asn Cys Asp Met Ser His Gly Gln
                370               375               380

Asp Cys Tyr Arg Gly Asn Gly Lys Asn Tyr Met Gly Asn Leu Ser Gln
385                 390               395                  400

Thr Arg Ser Gly Leu Thr Cys Ser Met Trp Asp Lys Asn Met Glu Asp
                405               410               415

Leu His Arg His Ile Phe Trp Glu Pro Asp Ala Ser Lys Leu Asn Glu
                420               425               430

Asn Tyr Cys Arg Asn Pro Asp Asp Asp Ala His Gly Pro Trp Cys Tyr
                435               440               445

Thr Gly Asn Pro Leu Ile Pro Trp Asp Tyr Cys Pro Ile Ser Arg Cys
                450               455               460

Glu Gly Asp Thr Thr Pro Thr Ile Val Asn Leu Asp His Pro Val Ile
465                 470               475                  480

Ser Cys Ala Lys Thr Lys Gln Leu Arg Val Val Asn Gly Ile Pro Thr
                485               490               495

Arg Thr Asn Ile Gly Trp Met Val Ser Leu Arg Tyr Arg Asn Lys His
                500               505               510

Ile Cys Gly Gly Ser Leu Ile Lys Glu Ser Trp Val Leu Thr Ala Arg
                515               520               525
```

```
Gln Cys Phe Pro Ser Arg Asp Leu Lys Asp Tyr Glu Ala Trp Leu Gly
        530              535              540

Ile His Asp Val His Gly Arg Gly Asp Glu Lys Cys Lys Gln Val Leu
545              550              555              560

Asn Val Ser Gln Leu Val Tyr Gly Pro Glu Gly Ser Asp Leu Val Leu
                 565              570              575

Met Lys Leu Ala Arg Pro Ala Val Leu Asp Asp Phe Val Ser Thr Ile
                 580              585              590

Asp Leu Pro Asn Tyr Gly Cys Thr Ile Pro Glu Lys Thr Ser Cys Ser
             595              600              605

Val Tyr Gly Trp Gly Tyr Thr Gly Leu Ile Asn Tyr Asp Gly Leu Leu
    610              615        .        620

Arg Val Ala His Leu Tyr Ile Met Gly Asn Glu Lys Cys Ser Gln His
625              630              635              640

His Arg Gly Lys Val Thr Leu Asn Glu Ser Glu Ile Cys Ala Gly Ala
                 645              650              655

Glu Lys Ile Gly Ser Gly Pro Cys Glu Gly Asp Tyr Gly Gly Pro Leu
             660              665              670

Val Cys Glu Gln His Lys Met Arg Met Val Leu Gly Val Ile Val Pro
        675              680              685

Gly Arg Gly Cys Ala Ile Pro Asn Arg Pro Gly Ile Phe Val Arg Val
    690              695              700

Ala Tyr Tyr Ala Lys Trp Ile His Lys Ile Ile Leu Thr Tyr Lys Val
705              710              715              720

Pro Gln Ser
    723
```

**Claims**

1.    A monoclonal antibody having affinity specific to an human anti-carcinoma protein termed TCF-II and a molecular weight of approximately 150,000 and belonging to subclass IgG$_1$.

2. A monoclonal antibody as claimed in claim 1, wherein an N-terminal amino acid sequence of an L chain of the monoclonal antibody is defined by either one of the following amino acid sequences:

Amino acid sequence (1):

Asp-Val-Val-Leu-Thr-Gln-Ser-Pro-Ala-
1    2    3    4    5    6    7    8    9

Thr-Leu-Ser-Val-Thr-Pro-Gly-Asp-Ser-
10   11   12   13   14   15   16   17   18

Val
19

Amino acid sequence (2):

Asp-Ser-Val-Leu-Thr-Gln-Ser-Pro-Ala-
1    2    3    4    5    6    7    8    9

Ser-Leu-Ala-Val-Ser-Leu-Gly-Gln-Arg-
10   11   12   13   14   15   16   17   18

Ala-Thr-Ile-Ser
19   20   21   22

Amino acid sequence (3):

Asp-Ile-Val-Leu-Thr-Gln-Ser-Pro-Val-
1    2    3    4    5    6    7    8    9

Thr-Leu-Ser-Val-Thr-Pro-Gly-Gly-Ser-
10   11   12   13   14   15   16   17   18

Val
19

3. A method for measuring a human anti-tumor protein, TCF-II, by using a monoclonal antibody having affinity specific to a human anti-tumor protein termed TCF-II and a molecular weight of approximately 150,000 and belonging to subclass IgG$_1$.

4. A method as claimed in claim 3, wherein said monoclonal antibody is defined by N-terminal amino acid sequence of L chain of the monoclonal antibodies comprising either one of the following amino acid sequences:

Amino acid sequence (1):

```
Asp-Val-Val-Leu-Thr-Gln-Ser-Pro-Ala-
 1   2   3   4   5   6   7   8   9

Thr-Leu-Ser-Val-Thr-Pro-Gly-Asp-Ser-
10  11  12  13  14  15  16  17  18

Val
19
```

Amino acid sequence (2):

```
Asp-Ser-Val-Leu-Thr-Gln-Ser-Pro-Ala-
 1   2   3   4   5   6   7   8   9

Ser-Leu-Ala-Val-Ser-Leu-Gly-Gln-Arg-
10  11  12  13  14  15  16  17  18

Ala-Thr-Ile-Ser
19  20  21  22
```

Amino acid sequence (3):

```
Asp-Ile-Val-Leu-Thr-Gln-Ser-Pro-Val-
 1   2   3   4   5   6   7   8   9

Thr-Leu-Ser-Val-Thr-Pro-Gly-Gly-Ser-
10  11  12  13  14  15  16  17  18

Val
19
```

5. A method as claimed in claim 3 or 4, wherein said monoclonal antibody is in a combination of a solid phase monoclonal antibody and an enzyme-labelled monoclonal antibody.

6. Use of a monoclonal antibody as claimed in claim 1 for diagnosis of a liver disease.

TABLE 1:   Sequence of Amino Acids of TCF-II

Met Trp Val Thr Lys Leu Leu Pro Ala Leu Leu Leu Gln His
                  5                        10

Val Leu Leu His Leu Leu Leu Pro Ile Ala Ile Pro Tyr
15                    20                       25

Ala Glu Gly Gln Arg Lys Arg Arg Asn Thr Ile His Glu Phe
        30                   35                    40

Lys Lys Ser Ala Lys Thr Thr Leu Ile Lys Ile Asp Pro Ala
            45                   50                    55

Leu Lys Ile Lys Thr Lys Lys Val Asn Thr Ala Asp Gln Cys
                 60                   65                       70

Ala Asn Arg Cys Thr Arg Asn Lys Gly Leu Pro Phe Thr Cys
                 75                   80

Lys Ala Phe Val Phe Asp Lys Ala Arg Lys Gln Cys Leu Trp
85                   90                   95

Phe Pro Phe Asn Ser Met Ser Ser Gly Val Lys Lys Glu Phe
        100                  105                  110

Gly His Glu Phe Asp Leu Tyr Glu Asn Lys Asp Tyr Ile Arg
        115                  120                  125

Asn Cys Ile Ile Gly Lys Gly Arg Ser Tyr Lys Gly Thr Val
            130                  135                  140

Ser Ile Thr Lys Ser Gly Ile Lys Cys Gln Pro Trp Ser Ser
            145                  150

Met Ile Pro His Glu His Ser Tyr Arg Gly Lys Asp Leu Gln
155              160              165

Glu Asn Tyr Cys Arg Asn Pro Arg Gly Glu Glu Gly Gly Pro
    170              175              180

Trp Cys Phe Thr Ser Asn Pro Glu Val Arg Tyr Glu Val Cys
        185              190              195

Asp Ile Pro Gln Cys Ser Glu Val Glu Cys Met Thr Cys Asn
            200              205              210

Gly Glu Ser Tyr Arg Gly Leu Met Asp His Thr Glu Ser Gly
            215              220

Lys Ile Cys Gln Arg Trp Asp His Gln Thr Pro His Arg His
225              230              235

Lys Phe Leu Pro Glu Arg Tyr Pro Asp Lys Gly Phe Asp Asp
    240              245              250

Asn Tyr Cys Arg Asn Pro Asp Gly Gln Pro Arg Pro Trp Cys
        255              260              265

Tyr Thr Leu Asp Pro His Thr Arg Trp Glu Tyr Cys Ala Ile
            270              275              280

Lys Thr Cys Ala Asp Asn Thr Met Asn Asp Thr Asp Val Pro
                285              290

Leu Glu Thr Thr Glu Cys Ile Gln Gly Gln Gly Glu Gly Tyr
295              300              305

Arg Gly Thr Val Asn Thr Ile Trp Asn Gly Ile Pro Cys Gln
    310              315              320

18

Arg Trp Asp Ser Gln Tyr Pro His Glu His Asp Met Thr Pro
            325             330             335

Glu Asn Phe Lys Cys Lys Asp Leu Arg Glu Asn Tyr Cys Arg
            340             345             350

Asn Pro Asp Gly Ser Glu Ser Pro Trp Cys Phe Thr Thr Asp
            355             360

Pro Asn Ile Arg Val Gly Tyr Cys Ser Gln Ile Pro Asn Cys
365             370             375

Asp Met Ser His Gly Gln Asp Cys Tyr Arg Gly Asn Gly Lys
    380             385             390

Asn Tyr Met Gly Asn Leu Ser Gln Thr Arg Ser Gly Leu Thr
    395             400             405

Cys Ser Met Trp Asp Lys Asn Met Glu Asp Leu His Arg His
            410             415             420

Ile Phe Trp Glu Pro Asp Ala Ser Lys Leu Asn Glu Asn Tyr
            425             430

Cys Arg Asn Pro Asp Asp Asp Ala His Gly Pro Trp Cys Tyr
435             440             445

Thr Gly Asn Pro Leu Ile Pro Trp Asp Tyr Cys Pro Ile Ser
    450             455             460

Arg Cys Glu Gly Asp Thr Thr Pro Thr Ile Val Asn Leu Asp
            465             470             475

His Pro Val Ile Ser Cys Ala Lys Thr Lys Gln Leu Arg Val
            480             485             490

Val Asn Gly Ile Pro Thr Arg Thr Asn Ile Gly Trp Met Val
495 500

Ser Leu Arg Tyr Arg Asn Lys His Ile Cys Gly Gly Ser Leu
505 510 515

Ile Lys Glu Ser Trp Val Leu Thr Ala Arg Gln Cys Phe Pro
520 525 530

Ser Arg Asp Leu Lys Asp Tyr Glu Ala Trp Leu Gly Ile His
535 540 545

Asp Val His Gly Arg Gly Asp Glu Lys Cys Lys Gln Val Leu
550 555 560

Asn Val Ser Gln Leu Val Tyr Gly Pro Glu Gly Ser Asp Leu
565 570

Val Leu Met Lys Leu Ala Arg Pro Ala Val Leu Asp Asp Phe
575 580 585

Val Ser Thr Ile Asp Leu Pro Asn Tyr Gly Cys Thr Ile Pro
590 595 600

Glu Lys Thr Ser Cys Ser Val Tyr Gly Trp Gly Tyr Thr Gly
605 610 615

Leu Ile Asn Tyr Asp Gly Leu Leu Arg Val Ala His Leu Tyr
620 625 630

Ile Met Gly Asn Glu Lys Cys Ser Gln His His Arg Gly Lys
635 640

Val Thr Leu Asn Glu Ser Glu Ile Cys Ala Gly Ala Glu Lys
645 650 655

Ile Gly Ser Gly Pro Cys Glu Gly Asp Tyr Gly Gly Pro Leu
      660                   665                   670

Val Cys Glu Gln His Lys Met Arg Met Val Leu Gly Val Ile
          675                   680                   685

Val Pro Gly Arg Gly Cys Ala Ile Pro Asn Arg Pro Gly Ile
              690                   695                   700

Phe Val Arg Val Ala Tyr Tyr Ala Lys Trp Ile His Lys Ile
                  705                   710

Ile Leu Thr Tyr Lys Val Pro Gln Ser
715                   720       723

Fig. 1

Fig. 2

Fig. 3

Fig. 4